Europäisches Patentamt

(19) European Patent Office

Office européen des brevets

(11) Numéro de publication: **0 077 345**
**B1**

(12) **FASCICULE DE BREVET EUROPEEN**

(45) Date de publication du fascicule du brevet:
08.01.86

(21) Numéro de dépôt: **82901129.5**

(22) Date de dépôt: **14.04.82**

(86) Numéro de dépôt international:
**PCT/FR 82/00071**

(87) Numéro de publication internationale:
**WO 82/03767 (11.11.82 Gazette 82/27)**

(51) Int. Cl.⁴: **A 61 F 5/37**

(54) **GILET ORTHOPEDIQUE DE SOUTIEN ET DE CONTENTION DANS LE TRAITEMENT DES TRAUMATISES ET DES OPERES DE L'EPAULE, DE LA CEINTURE SCAPULAIRE ET DU MEMBRE SUPERIEUR.**

(30) Priorité: **28.04.81 FR 8108625**
**14.12.81 FR 8123781**

(43) Date de publication de la demande:
**27.04.83 Bulletin 83/17**

(45) Mention de la délivrance du brevet:
**08.01.86 Bulletin 86/2**

(84) Etats contractants désignés:
**AT BE CH DE FR GB LI LU NL SE**

(56) Documents cités:
**CH - A - 603 153**
**GB - A - 1 291 780**
**US - A - 1 745 446**
**US - A - 2 549 703**
**US - A - 2 560 243**
**US - A - 3 515 131**

(73) Titulaire: **BERREHAIL, Mohamed, Immeuble Royal,
F-38860 Les-Deux-Alpes (FR)**

(72) Inventeur: **BERREHAIL, Mohamed, Immeuble Royal,
F-38860 Les-Deux-Alpes (FR)**

(74) Mandataire: **Maureau, Pierre, Cabinet GERMAIN &
MAUREAU Le Britannia - Tour C 20, Boulevard E.
Déruelle, F-69003 Lyon (FR)**

ACTORUM AG

**Description**

La présente invention concerne un gilet orthopédique de soutien et de contention dans le traitement des traumatisés et des opérés de l'épaule, de la ceinture scapulaire et du membre supérieur.

On a déjà proposé de nombreux dispositifs visant à réaliser le soutien et la contention de cette partie supérieure du corps. Il s'agit le plus souvent de bandages qui présentent de multiples inconvénients, qu'il s'agisse d'un manque fréquent de solidité et de résistance du bandage lui-même, ou des difficultés de son maintien qui obligent à le refaire fréquemment. Quand ces bandages sont d'usage unique, leur prix de revient est élevé. On a pu également constater des réactions d'intolérance cutanée dans le cas de l'emploi de certains systèmes adhésifs. Ces bandages ne respectent pas en général le confort du malade puisqu'il n'est pratiquement pas possible de réaliser une toilette convenable ni d'apporter des soins locaux sans les défaire ou les détruire. Enfin le coude, qui n'est souvent pas concerné par le traumatisme ou l'intervention, se trouve fréquemment bloqué par le bandage et devient le siège de douleurs rapportées et d'ankyloses parfois intolérables.

Le document US-A-2549703 décrit un gilet orthopédique de soutien et de contention de l'épaule, de la ceinture scapulaire et du membre supérieur, composé essentiellement de quatre panneaux solidaires.

Sur ce gilet connu, un premier panneau est destiné à enserrer une partie du thorax, un deuxième panneau est destiné à enserrer la partie avant du thorax, la partie axillaire et la partie antérieure de l'épaule et du bras, et se prolonge latéralement par un troisième panneau destiné à enserrer la partie postérieure de l'épaule et du bras, et à y être maintenu en place, ledit deuxième panneau étant prolongé à sa partie inférieure par un quatrième panneau destiné à servir de reposoir à l'avant-bras, lesdits panneaux étant équipés sur leurs deux faces de moyens de fermeture et serrage progressif permettant au gilet d'être indifféremment utilisé à gauche ou à droite par simple retournement. Ce gilet présente, en outre, une ouverture pour le passage du bras malade.

Les gilets de ce type présentent toutefois de nombreux inconvénients; l'immobilisation du membre malade nécessite qu'ils recouvrent tout le haut du thorax, y compris l'épaule non malade. La fermeture et le réglage du serrage de ces gilets se fait au moyen de nœuds effectués sur des lacets ce qui cause un retrait progressif du bandage vers le nœud et est un obstacle à un réglage et au maintien uniforme sur toute la largeur du gilet. Il s'agit de plus d'un mode de fermeture relativement long et qui peut être facilement défait par les enfants ou les malades agités quand les nœuds se trouvent à portée de leur main valide puisque la fermeture de ces gilets s'ouvre sur le devant.

La présente invention s'est donné pour but de pallier ces inconvénients en proposant un gilet orthopédique qui permet une immobilisation et une contention confortables et règlables de l'épaule, de la ceinture scapulaire et du membre supérieur des traumatisés et opérés. Il peut être placé facilement et peut s'enlever à tout moment pour permettre une intervention locale sporadique: toilette, pansement, ablation de fils de suture, application d'une thérapeutique locale.

C'est ainsi que le gilet orthopédique se soutien et de contention de l'épaule, de la ceinture scapulaire et du membre supérieur correspondant, comporte, selon l'invention:

– un premier panneau (2) destiné à recouvrir la partie arrière du thorax du porteur du gilet;

– un deuxième panneau (3) qui prolonge latéralement le premier panneau (2) et est destiné à recouvrir la partie avant du thorax, la partie axillaire et la partie antérieure de l'épaule et du bras à soutenir;

–un troisième panneau (4);

– un quatrième panneau (5), qui prolonge partiellement la partie inférieure du deuxième panneau (3) et est destiné à servir de reposoir à l'avant-bras du porteur du gilet;

– des moyens de fermeture et de serrage progressif (8–12) équipant lesdits panneaux sur leurs deux faces et permettant d'utiliser le gilet indifféremment à gauche ou à droite par simple retournement et,

– une ouverture (38, 39) pour le passage du bras malade ménagée entre les deuxième et troisième panneaux (3, 4).

Il est caractérisé en ce que:

– le deuxième panneau (3) est conformé de manière à recouvrir à lui seul la quasi-totalité de la partie avant du thorax du porteur du gilet;

– le troisième panneau (4) prolonge latéralement le deuxième panneau (3) du côté opposé au premier panneau (2) et,

– le troisième panneau (4) est conformé de manière à pouvoir enserrer la partie postérieure de l'épaule et du bras malade et à recouvrir au moins une partie du premier panneau (2) pour le maintenir en place sur la partie arrière du thorax assurant ainsi la fermeture du gilet dans le dos du porteur.

Selon un mode de réalisation préféré de l'invention, le premier panneau est sensiblement rectangulaire et est prolongé latéralement par le deuxième panneau délimité, à sa partie inférieure, par une ligne sensiblement horizontale prolongeant le bord inférieur du premier panneau, à sa partie supérieure par une courbe concave ascendante et sur le côté opposé au premier panneau par une courbe convexe descendante prolongée par une ligne sensiblement verticale, ledit deuxième panneau étant rendu solidaire à cet endroit, par couture ou tout autre moyen adapté, du troisième panneau dont le bord d'assemblage présente le même profil, ledit troisième panneau étant en outre délimité, à sa partie supérieure, par une ligne oblique, à sa partie inférieure par une ligne sensiblement horizontale prolongeant le bord inférieur des premier et deuxième panneaux et sur son côté libre par une ligne sensiblement verticale; le quatrième panneau présente une forme sensiblement rectangulaire et est solidaire par un de ses plus grands côtés de la partie inférieure du deuxième panneau.

Selon un mode de réalisation de l'invention, la ligne de solidarisation entre les deuxième et troisième panneaux est interrompue à sa partie inférieure ménageant ainsi une fente permettant le passage de l'avant-bras.

Selon un autre mode de réalisation, la ligne de solidarisation entre le deuxième et le troisième panneaux est provisoirement interrompue et une échancrure est ménagée de part et d'autre de cette interruption, permettant la réalisation d'une ouverture permettant le passage de l'avant-bras. Les trois panneaux formant le corps du gilet peuvent se prolonger jusqu'à la ceinture du porteur; le quatrième panneau, servant de reposer à l'avant-bras, est alors rendu solidaire du deuxième panneau au niveau d'une fente ménagée dans ce dernier un peu au-dessus de la ceinture; le reposoir peut ainsi glisser d'un côté ou de l'autre du gilet, contribuant à sa réversibilité. La partie inférieure des trois premiers panneaux comporte alors avantageusement des moyens de fixation sur le pantalon ou la ceinture.

Selon un autre mode de réalisation, les trois panneaux s'arrêtent avant la ceinture du porteur et le quatrième panneau est solidaire du deuxième panneau au niveau du bord inférieur de celui-ci. Les différents panneaux constituant le corps du gilet sont réalisés en une matière leur permettant d'exercer par eux-mêmes une certaine contention du porteur.

Selon une caractéristique de l'invention, cette matière est un tissu ou un tricot élastique.

Selon une autre caractéristique, la matière constituant le corps du gilet est un assemblage de feuilles imperméables réunies entre elles de façon étanche, et les différents panneaux peuvent être gonflés par introduction d'un fluide quelconque et notamment de l'air.

Les différents panneaux peuvent être gonflés par un système de dépression, du genre couramment dénommé matelas-coquille.

Il a paru en outre intéressant de perfectionner le gilet selon l'invention en l'équipant de moyens permettant tant de protéger le coude du patient que de maintenir l'avant-bras en place et de l'empêcher de glisser hors du gilet, ainsi que de moyens permettant d'exercer à volonté une traction plus ou moins forte de l'épaule vers l'arrière, et de maintenir l'épaule en position de rotation externe.

Selon une forme de réalisation préférée de l'invention, le gilet est équipé d'une languette amovible ou semi-amovible pourvue de moyens propres d'attache prévus pour coopérer avec d'autres moyens placés sur ledit gilet permettant d'assurer la protection du coude et le maintien de l'avant-bras à l'intérieur du gilet, cette languette étant éventuellement associée à une autre languette amovible disposée au niveau de l'épaule où elle est maintenue par des moyens propres d'attache et permettant d'une part d'exercer à volonté une traction plus ou moins forte de l'épaule vers l'arrière, et d'autre part, de maintenir l'épaule en position de rotation externe grâce à des moyens de fixation règlables prévus sur ledit gilet et susceptibles de coopérer avec les moyens propres d'attache de ladite languette.

Les languettes sont réalisées de préférence en une matière élastique, éventuellement de mêmes nature et composition que celle ayant servi à réaliser le corps du gilet.

La présente invention sera mieux comprise d'ailleurs et ses avantages ressortiront bien de la description qui suit, en référence au dessin schématique annexé dans lequel:

— les figures 1, 2 et 3 sont des vues en plan des différentes formes du gilet selon l'invention;

— les figures 4 à 11 représentent les différentes étapes de l'enfilage puis de la mise en place d'un gilet représenté à la figure 1;

— la figure 12 est une vue en coupe.

Sur les figures (1) désigne de façon générale le gilet, (2), (3), (4) et (5) représentent respectivement les premier, deuxième, troisième et quatrième panneaux constitutifs du gilet, (6) la longuette prévue pour assurer la protection du coude et le maintien de l'avant-bras et (7) la languette prévue pour exercer une traction sur l'épaule puis pour maintenir cette dernière en position disirée.

Les différents panneaux et les deux languettes sont munis de moyens (8), (9), (10), (11), (12), (13), (14), (15), (16), (17), (18), (19), permettant d'assurer par engagement réciproque, leur attache, leur serrage et leur fixation.

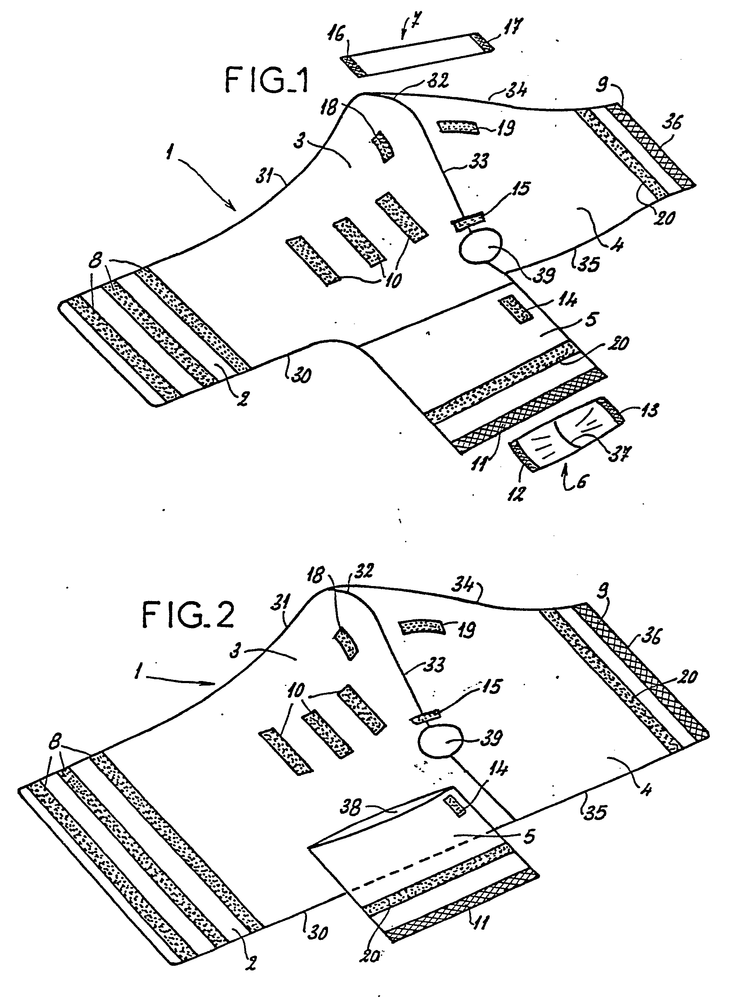

Comme on le voit sur la figure 1, le gilet (1) selon l'invention se compose essentiellement des quatre panneaux solidaires (2), (3), (4) et (5).

Le premier panneau (2) est sensiblement rectangulaire et est prolongé latéralement par le deuxième panneau (3) délimité, à sa partie inférieure, par une ligne (30) sensiblement horizontale, prolongeant le bord inférieur du panneau (2); il est également délimité à sa partie supérieure, par une courbe concave ascendante (31) et sur le côté opposé au panneau (2) par une courbe convexe desdendante (32) prolongée par une ligne sensiblement verticale (33); le panneau (3) est rendu solidaire, au niveau des courbe et ligne (32) et (33) du panneau (4). Cette solidarisation peut être effectuée par couture, par soudure ou par toute autre moyen convenable adapté à la nature de la matière constituant le gilet. Ce panneau (4), dont le bord d'assemblage présente le même profil que celui du bord du panneau (3) auquel il est assemblé, est délimité, à sa partie supérieure, par une ligne oblique (34) et à sa partie inférieure par une ligne sensiblement horizontale (35) prolongeant le bord inférieur des panneaux (2) et (3). Il est limité, sur son côté libre, par une ligne sensiblement verticale (36). Le panneau (5), destiné à servir de reposoir pour le bras présente une forme sensiblement rectangulaire et est solidaire, par un de ses plus grands côtés, de la partie inférieure du panneau (3). La languette (6) prévue pour assurer la protection du coude et le maintien de l'avant-bras est essentiellement rectangulaire. Elle est avantageusement galbée en (37) afin de lui permettre de mieux épouser la forme du coude.

La languette (7) prévue pour exercer une traction sur l'épaule puis pour maintenir celle-ci dans la position désirée est rectangulaire.

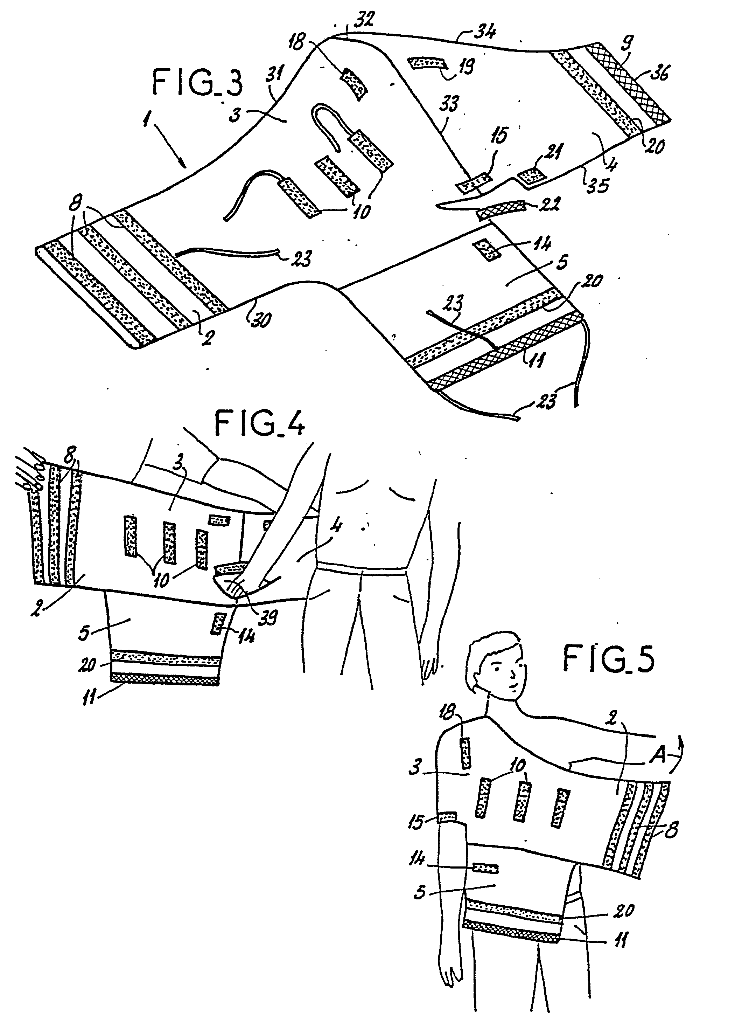

Dans le mode de réalisation représenté aux figures 1 et 3, l'ensemble du gilet est prévu pour s'arrêter au-dessus de la ceinture et le reposoir (5) est rendu solidaire du panneau (3) au niveau du bord inférieur de celui-ci.

Dans le mode de réalisation représenté à la figure 2, les panneaux (2), (3) et (4) formant le corps du gilet (1) se prolongent jusqu'à la ceinture, et une fente (38) est ménagée dans le panneau (3) pour permettre le passage du reposoir (5), solidaire dudit panneau (3) d'un côté ou de l'autre du gilet (1), selon que celui-ci est utilisé à gauche ou à droite. Des moyens de fixation (non représentés au dessin) peuvent alors être prévus à la partie inférieure des panneaux (2), (3) et (4) pour coopérer avec des moyens complémentaires ménagés sur le pantalon ou la ceinture.

Dans le mode de réalisation représenté aux figures 1 et 2, la ligne de solidarisation prévue entre les panneaux (3) et (4) est provisoirement interrompue au niveau du dessous de bras et une échancrure (39) est ménagée de part et d'autre pour permettre le passage du bras.

Dans le mode de réalisation représenté à la figure 3, les panneaux (3) et (4) ne sont rendus solidaires que jusqu'au niveau du dessous de bras et des moyens de fixation complémentaires (21) et (22) sont prévus pour rendre les panneaux (3) et (4) solidaires au niveau du dessous de bras.

Les différentes étapes d'enfilage et de mise en place du gilet (1), du reposoir (5) et des languettes (6) et (7) vont maintenant être décrites en détail.

Comme on le voit sur la figure 4, le gilet s'enfile comme un gilet ordinaire en faisant passer le bras dans l'ouverture (39) ménagée à cet effet.

Pour le gilet correspondant au mode de réalisation représenté à figure 3 la mise en place serait encore facilitée puisqu'il suffit dans ce cas de recouvrir l'épaule intéressée du corps du gilet déplié, puis d'assurer, sans avoir à déplacer le bras ou l'épaule blessée, sa fermeture au niveau du dessous de bras, par les moyens d'attache et de fixation (21) et (22); dans ce mode de réalisation, plus spécialement prévu pour les secours d'urgence, des moyens de fixation supplémentaires, qui peuvent être des lacettes, généralement désignées par (23), sont également prévus pour faciliter la mise en place du gilet sur le lieu de l'accident.

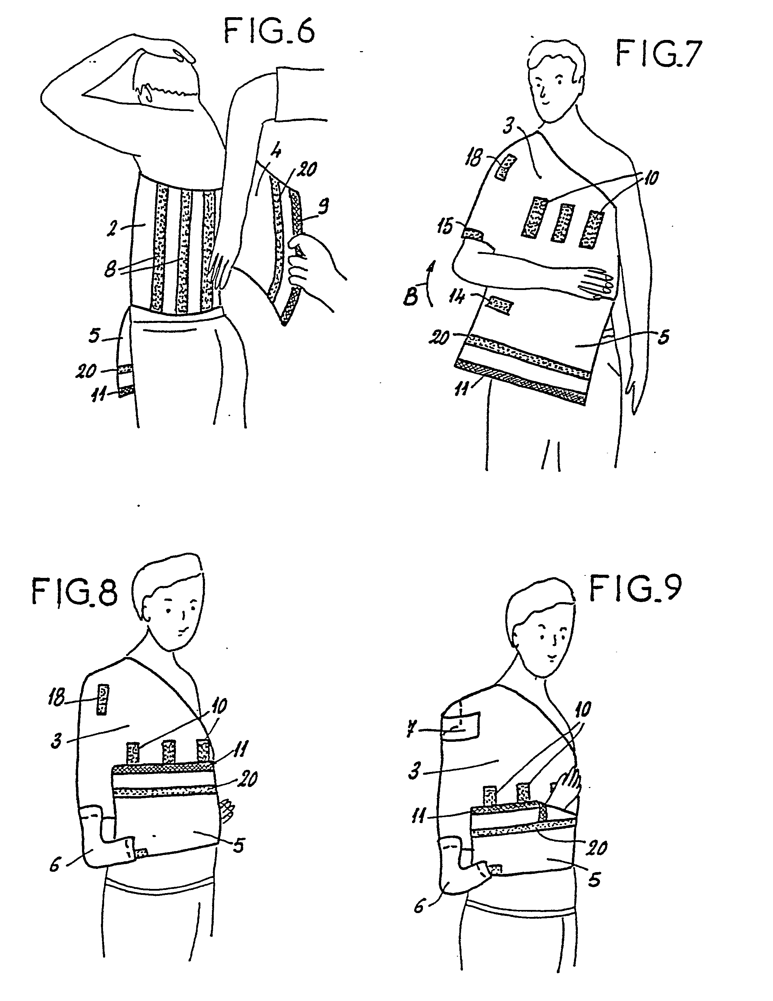

Une fois le gilet enfilé, on enveloppe (figure 5) la zone intéressée (épaule, clavicule, omoplate), puis comme indiqué par la flèche A, on referme le corps (1) du gilet au niveau de la région axillaire opposée en faisant coopérer les moyens d'attache (8) et (9). Ces moyens réglables, ainsi que la nature élastique de la matière constituant le corps du gilet, permettent de bien mouler le thorax du patient et d'assurer une contention et une immobilisation plus ou moins ferme (figure 6).

Dans une troisième étape, représenté à la figure 7, on rabat le reposoir (5) comme indiqué par la flèche B et on le fixe au corps (1) du gilet en faisant coopérer les moyens d'attache réglables (10) et (11), l'avant-bras du patient étant replié de préférence selon un angle d'environ 90°.

Deux positions différentes de l'avant-bras sont représentées aux figures 8, 9 et 10.

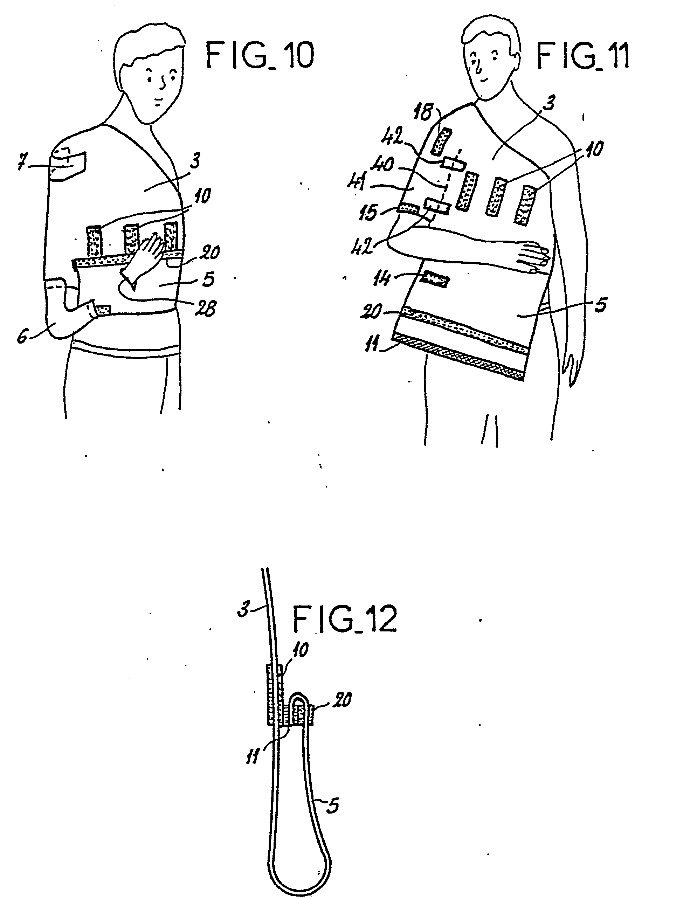

A la figure 10, une fente (28) est ménagée dans le reposoir (5) pour permettre à la main de sortir. On peut à ce stade, et si l'état du patient l'exige, intercaler un bloc de mousse ou de coton sous l'avant-bras ou le bras. On assure ensuite la protection du coude et le maintien en place de l'avant-bras en plaçant la languette (6) contre le coude et en assurant sa fixation grâce à la coopération des moyens (12) et (13) aux moyens (14) et (15) placés respectivement sur la languette (6), sur le reposoir (5) et sur le corps du gilet (1) (figure 8). La languette (6) est réalisée de préférence en une matière élastique de même nature que la matière constituant le corps du gilet (1) et le reposoir (5). Comme dit précédemment, cette languette (6) est avantageusement galbée de façon à mieux épouser la forme du coude qu'elle soutient par sa partie inférieure. La languette (6) peut être également fixée de façon permanente par une de ses extrémités soit sur le reposoir (5), soit sur le corps (1) du gilet. Enfin, et comme on le voit à la figure (9), on met en place la languette (7) en faisant coopérer les moyens (16) et (17) disposés sur ladite languette avec les moyens (18) et (19) prévus sur les deux côtés du corps (1) du gilet. Il est ainsi possible, notamment grâce à la position transversale du moyen (19) d'exercer une traction plus ou moins forte de l'épaule vers l'arrière, puis de maintenir cette dernière en position de rotation externe. Cette languette (7) est également réalisée de préférence en une matière élastique, éventuellement similaire à celle constituant le corps (1) du gilet et le reposoir (5).

Les divers moyens d'attache et de fixation (8), (9), (10), (11), (14), (15), (18) et (19) sont disposés sur les deux faces du corps (1) du gilet et du reposoir (5) afin de permettre la réalisation simple d'un gilet réversible permettant une utilisation gauche ou droite par simple retournement. Ces moyens de fixation ainsi que les moyens de fixation (12), (13), (16) et (17) prévus sur les languettes peuvent être de tout type connu susceptible de coopérer et éventuellement du type velours et crochet tels que ceux commerciales sous la marque VELCRO.

On peut avantageusement prévoir dans ce cas un moyen de fixation supplémentaire «velours», tel que celui généralement représenté en (20) sur la figure 10, afin de coopérer avec un des moyens «crochet» ceci pour éviter toute rugosité gênante à l'extérieur dudit gilet (figure 12).

Sur le mode de réalisation représenté à la figure 11 il est ménagé, par exemple à l'aide d'une couture (40), un système (41) de manche, externe ou interne, afin de faciliter la protection de la région axillaire et de contribuer au confort du porteur. Ce système de manche est bien évidemment équipé lui aussi de système propre de réversibilité (42). Enfin il peut être prévu sur le reposoir (5) plusieurs bandes de fixation (11) afin d'adapter ledit reposoir à la conformation du bras à soutenir.

Le gilet orthopédique selon l'invention constitue donc un moyen nouveau d'immobilisation et de contention du membre supérieur, de l'épaule et de la ceinture scapulaire. Sa réversibilité et son extensibilité permettent de l'utiliser à volonté à gauche ou à droite et de l'adapter à toutes les morphologies. En traumatologie d'urgence, il sert enfin d'attelle d'emballage.

**Revendications**

1. Gilet orthopédique de soutien et de contention de l'épaule, de la ceinture scapulaire et du membre supérieur correspondant, comportant,
– un premier panneau (2) destiné à recouvrir la partie arrière du thorax du porteur du gilet;
– un deuxième panneau (3) qui prolonge latéralement le premier panneau (2) et est destiné à recouvrir la partie avant du thorax, la partie axillaire et la partie antérieure de l'épaule et du bras à soutenir;
– un troisième panneau (4);
– un quatrième panneau (5) qui prolonge partiellement la partie inférieure du deuxième panneau (3) et est destiné à servir de reposoir à l'avant-bras du porteur du gilet;
– des moyens de fermeture et de serrage progressif (8, 12) équipant lesdits panneaux sur leurs deux faces et permettant d'utiliser le gilet indifféremment à gauche ou à droite par simple retournement et,
– une ouverture (38, 39) pour le passage du bras malade ménagée entre les deuxième et troisième panneaux (3, 4), caractérisé en ce que:
– le deuxième panneau (3) est conformé de manière à recouvrir à lui seul la quasi-totalité de la partie avant du thorax du porteur du gilet;
– le troisième panneau (4) prolonge latéralement le deuxième panneau (3) du côté opposé au premier panneau (2) et en ce que,
– le troisième panneau (4) est conformé de manière à pouvoir enserrer la partie postérieure de l'épaule et du bras malade et à recouvrir au moins une partie du premier panneau (2) pour le maintenir en place sur la partie arrière du thorax assurant ainsi la fermeture du gilet dans le dos du porteur.

2. Gilet orthopédique selon la revendication 1, caractérisé en ce que le premier panneau (2) est sensiblement rectangulaire et est prolongé latéralement par le deuxième panneau (3) délimité, à sa partie inférieure, par une ligne sensiblement horizontale (30) prolongeant le bord inférieur du premier panneau, à sa partie supérieure par une courbe concave ascendante (31) et sur le côté opposé au premier panneau par une courbe convexe (32) descendante prolongée par une ligne sensiblement verticale (33), ledit deuxième panneau étant rendu solidaire à cet endroit, par une couture ou tout autre moyen adapté, du troisième panneau (4) dont le bord d'assemblage présente le même profil, ledit troisième panneau étant en outre délimité, à sa partie supérieure, par une ligne oblique (34), à sa partie inférieure par une ligne sensiblement horizontale (35) prolongeant le bord inférieur des premier et deuxième panneaux et sur son côté libre par une ligne sensiblement verticale (36), et que le quatrième panneau (5) présente une forme sensiblement rectangulaire et est solidaire par un de ses plus grands côtés de la partie inférieure du deuxième panneau.

3. Gilet orthopédique selon les revendications 1 et 2 caractérisé en ce que la ligne de solidarisation entre le deuxième et le troisième panneau est interrompue à sa partie inférieure ménageant ainsi une fente permettant le passage de l'avant-bras.

4. Gilet orthopédique selon les revendications 1 et 2, caractérisé en ce que la ligne de solidarisation entre le deuxième et le troisième panneau est provisoirement interrompue et une échancrure est ménagée de part et d'autre de cette interruption permettant la réalisation d'une ouverture (39) permettant le passage de l'avant-bras.

5. Gilet orthopédique selon l'une quelconque des revendications 1 à 4, caractérisé en ce que les panneaux (2), (3) et (4) formant le corps du gilet (1) se prolongent jusqu'à la ceinture du porteur et le reposoir (5) est rendu solidaire du panneau (3) au niveau d'une fente (38) ménagée dans ledit panneau (3) au-dessus de la ceinture.

6. Gilet orthopédique selon la revendication 5 caractérisé en ce que des moyens de fixation sont prévus à la partie inférieure des panneaux (2), (3) et (4) pour coopérer avec des moyens complémentaires ménagés sur le pantalon ou la ceinture.

7. Gilet orthopédique selon l'une quelconque des revendications 1 à 6 caractérisé en ce qu'il est équipé d'une languette amovible ou semi-amovible (6) pourvue de moyens propres d'attache (12) et (13) prévus pour coopérer avec d'autres moyens (14) et (15) placés sur ledit gilet, permettant d'assurer le soutien et la protection de la partie inférieure du coude et le maintien de l'avant-bras à l'intérieur du gilet.

8. Gilet orthopédique selon l'une quelconque des revendications 1 à 7, caractérisé en ce qu'il est équipé d'une autre languette (7) amovible ou semi-amovible disposée au niveau de l'épaule où elle est maintenue par des moyens propres d'attache (16) et (17) et permettant d'une part d'exercer à volonté une traction plus ou moins forte de l'épaule vers l'arrière, et d'autre part de maintenir l'épaule en position de rotation externe grâce à des moyens de fixation réglables (18) et (19) prévus sur ledit gilet et susceptibles de coopérer avec les moyens propres d'attache de ladite languette, ces moyens étant disposés en position facilitant leur mise en place.

9. Gilet orthopédique selon l'une quelconque des revendications 1 à 8, caractérisé en ce que les différents panneaux constituant le corps du gilet (1) sont réalisés en une matière permettant d'exercer par elle-même une certaine contention.

10. Gilet orthopédique selon la revendication 9 caractérisé en ce qu'il est réalisé en un tissu ou un tricot élastique.

11. Gilet orthopédique selon la revendication 9 caractérisé en ce qu'il est constitué par un assemblage de feuilles imperméables réunies entre elles de façon étanche, et peut être gonflé par introduction d'un fluide.

12. Gilet orthopédique selon la revendication 9 caractérisé en ce qu'il est constitué par un assemblage de feuilles imperméables et peut être gonflé par un système de dépression.

13. Gilet orthopédique selon les revendications 7 et 8, caractérisé en ce que les languettes sont réalisées en une matière élastique, éventuellement de même composition que celle constituant le corps du gilet.

14. Gilet orthopédique selon l'une quelconque des revendications 1 à 13, caractérisé en ce que la ligne de solidarisation prévue entre les panneaux (3) et (4) est provisoirement interrompue au niveau du dessous de bras et une échancrure (39) est ménagée de part et d'autre pour permettre le passage du bras.

15. Gilet orthopédique selon l'une quelconque des revendications 1 à 13, caractérisé en ce que les panneaux (3) et (4) ne sont rendus solidaires que jusqu'au niveau du dessous de bras et des moyens de fixation complémentaires (21) et (22) sont prévus pour rendre les panneaux (3) et (4) solidaires au niveau du dessous de bras.

16. Gilet orthopédique selon la revendication 15, caractérisé en ce que les moyens de fixation supplémentaires (23) sont prévus pour faciliter l'utilisation du gilet comme attelle d'emballage.

17. Gilet orthopédique selon l'une quelconque des revendications 1 à 16, caractérisé en ce qu'au moins une partie des moyens d'attache (8), (9), (10), (11), (12), (13), (14), (15), (16), (17), (18) et (19) sont disposés dans le sens horizontal.

18. Gilet orthopédique selon l'une quelconque des revendications 1 à 16, caractérisé en ce qu'au moins une partie des moyens d'attache (8), (9), (10), (11), (12), (13), (14), (15), (16), (17), (18) et (19) sond disposés dans le sens vertical.

19. Gilet orthopédique selon l'une quelconque des revendications 1 à 16, caractérisé en ce qu'au moins une partie des moyens d'attache (8), (9), (10), (11), (12), (13), (14), (15), (16), (17), (18) et (19) sont disposés dans le sens oblique.

20. Gilet orthopédique selon l'une quelconque des revendications 1 à 19, caractérisé en ce que les moyens d'attache et de fixation sont du type velours et crochet.

21. Gilet orthopédique selon la revendication 20 caractérisé en ce qu'un moyen complémentaire type velours (20) est prévu afin de coopérer avec les moyens du type crochet apparaissant à la surface du gilet.

22. Gilet orthopédique selon l'une quelconque des revendications 1 à 21, caractérisé en ce qu'il comporte un système de manche (41) externe ou interne au gilet comportant son système propre de réversibilité et de serrage (42).

23. Gilet orthopédique selon l'une quelconque des revendications 1 à 22, caractérisé en ce que le reposoir est équipé de plusieurs moyens de fixation (11) juxtaposés de manière à permettre de l'adapter à la taille du bras à soutenir.

24. Gilet orthopédique selon l'une quelconque des revendications 1 à 23, caractérisé en ce qu'une fente (28) est ménagée dans le reposoir (5) pour permettre la sortie de la main.

**Patentansprüche**

1. Orthopädische Weste zum Stützen und Zusammenhalten der Schulter, des Schultergürtels und des entsprechenden oberen Gliedmasses
— mit einem ersten Feld (2) zur Abdeckung des hinteren Brustkorbbereichs des Trägers der Weste,
— mit einem zweiten Feld (3), das in seitlicher Verlängerung des ersten Feldes (2) angeordnet ist und zur Abdeckung des vorderen Brustkorbbereichs, des Achselbereichs und des vorderen Schulterbereichs sowie des zu stützenden Arms dient,
— mit einem dritten Feld (4),
— mit einem vierten Feld (5), das den unteren Teil des zweiten Feldes (3) teilweise verlängert und als Armschlinge für den Unterarm des Trägers der Weste (1) dient,
— mit Mitteln (8, 12) zum Schliessen und progressiven Spannen, die beidseitig der Felder angeordnet sind und die es ermöglichen, die Weste durch einfaches Umwenden sowohl linksseitig als auch rechtsseitig zu benutzen,
— sowie mit einer Öffnung (38, 39) zwischen dem zweiten und dem dritten Feld (3, 4) für den Durchtritt des kranken Arms, dadurch gekennzeichnet,
— dass das zweite Feld (3) derart ausgebildet ist, dass es allein fast die gesamte Vorderseite des Brustkorbes des Trägers der Weste abdeckt,
— dass das dritte Feld (4) das zweite Feld (3) auf der dem ersten Feld (2) entgegengesetzten Seite seitlich verlängert,
— und dass das dritte Feld (4) derart ausgebildet ist, dass es die Rückseite der Schulter und des kranken Arms umschliesst und wenigstens einen Teil des ersten Feldes (2) überdecken kann, um letzteres auf der Rückseite des Brustkorbs an seiner vorgesehenen Stelle zu halten und so das Schliessen der Weste im Rücken des Trägers zu ermöglichen.

2. Orthopädische Weste nach Anspruch 1, dadurch gekennzeichnet, dass das erste Feld (2) annähernd Rechteckform besitzt und seitlich durch das zweite Feld (3) verlängert ist, dass dieses zweite Feld (3) an seinem unteren Rand von einer den unteren Rand des ersten Feldes forsetzenden annähernd horizontalen Linie (30), an seinem oberen Rand durch eine ansteigende konkave Kurve (31) und an der dem ersten Feld (2) entgegengesetzten Seite von einer abfallenden konvexen Kurve (32) begrenzt ist, die sich in einer annähernd vertikalen Linie (33) fortsetzt, dass das zweite Feld (3) an dieser Stelle durch eine Naht oder ein anderes geeignetes Mittel mit dem dritten Feld (4) fest verbunden ist, dessen Verbindungsrand das gleiche Profil aufweist, dass das dritte Feld (4) ausserdem an seinem oberen Rand durch von einer schiefen Linie (34), an seinem unteren Rand durch eine den unteren Rand des ersten und des zweiten Feldes fortsetzende, annähernd horizontale Linie (35) und an seiner freien Seite von einer annä-

herend vertikalen Linie (36) begrenzt ist, und dass das vierte Feld (5) eine im wesentlichen rechteckige Form besitzt und mit einer seiner grösseren Seiten fest mit dem unteren Teil des zweiten Feldes verbunden ist.

3. Orthopädische Weste nach Anspruch 1 oder 2, dadurch gekennzeichnet, dass die Verbindungslinie zwischen dem zweiten und dem dritten Feld in ihrem unteren Teil unterbrochen ist und so einen Schlitz für den Durchtritt des vorderen Arms bildet.

4. Orthopädische Weste nach Anspruch 1 oder 2, dadurch gekennzeichnet, dass die Verbindungslinie zwischen dem zweiten und dem dritten Feld provisorisch unterbrochen ist, und dass zu beiden Seiten dieser Unterbrechung ein Ausschnitt vorgesehen ist, der die Herstellung einer Öffnung (39) für den Durchtritt des vorderen Arms ermöglicht.

5. Orthopädische Weste nach einem der Ansprüche 1 bis 4, dadurch gekennzeichnet, dass das erste, das zweite und das dritte Feld (2, 3, 4), die den Körper der Weste (1) bilden, bis zur Gürtellinie des Trägers reichen, und dass das die Armschlinge (5) bildende vierte Feld (5) mit dem zweiten Feld (3) in Höhe eines in diesem (3) über der Gürtellinie ausgebildeten Schlitzes (38) fest verbunden ist.

6. Othopädische Weste nach Anspruch 5, dadurch gekennzeichnet, dass im unteren Teil der Felder (2, 3, 4) Befestigungsmittel vorgesehen sind, die mit an der Hose oder an dem Gürtel angebrachten komplementären Befestigungsmitteln zusammenwirken.

7. Orthopädische Weste nach einem der Ansprüche 1 bis 6, dadurch gekennzeichnet, dass sie eine lösbare oder halblösbare Zunge (6) aufweist, die mit eigenen Befestigungsmitteln (12, 13) ausgestattet ist, die im Zusammenwirken mit anderen an der Weste vorgesehenen Befestigungsmitteln (14, 15) die Stützung und den Schutz der Unterseite des Ellbogens und die Halterung des vorderen Arm im Innern der Weste ermöglichen.

8. Orthopädische Weste nach einem der Ansprüche 1 bis 7, dadurch gekennzeichnet, dass eine weitere lösbare oder halblösbare Zunge (7) in Höhe der Schulter angeordnet und dort durch eigene Befestigungsmittel (16, 17) gehalten ist, die einerseits auf Wunsch die Ausübung eines mehr oder weniger starken Zuges der Schulter nach hinten ermöglichen und andererseits mit Hilfe von an der Weste vorgesehene verstellbaren Befestigungsmitteln (18, 19), die mit den eigenen Befestigungsmitteln der Zunge (7) zusammenwirken, die Halterung der Schulter in einer nach aussen gedrehten Position ermöglichen, wobei die Mittel in einer Position angeordnet sind, die ihr Ansetzen erleichtern.

9. Orthopädische Weste nach einem der Ansprüche 1 bis 8, dadurch gekennzeichnet, dass die verschiedenen den Körper der Weste (1) bildenden Felder aus einem Material bestehen, das von sich aus eine gewisse Haltekraft auszuüben vermag.

10. Orthopädische Weste nach Anspruch 9, dadurch gekennzeichnet, dass sie aus einem Gewebe oder Gewirke oder einem elastischen Trikotstoff besteht.

11. Orthopädische Weste nach Anspruch 9, dadurch gekennzeichnet, dass sie aus undurchlässigen, dicht miteinander verbundenen Folien zusammengesetzt und durch Einführung eines Fluids aufblasbar ist.

12. Orthopädische Weste nach Anspruch 9, dadurch gekennzeichnet, dass sie aus undurchlässigen Folien zusammengesetzt ist und durch ein Unterdrucksystem aufblasbar ist.

13. Orthopädische Weste nach Anspruch 7 und 8, dadurch gekennzeichnet, dass die Zungen aus elastischem Material bestehen, das vorzugsweise die gleiche Zusammensetzung hat wie das Material des Körpers der Weste.

14. Orthopädische Weste nach einem der Ansprüche 1 bis 13, dadurch gekennzeichnet, dass die Verbindungslinie zwischen dem zweiten Feld (3) und dem dritten Feld (4) in Höhe des vorderen Arms provisorisch unterbrochen ist und dass zu beiden Seiten eine Aussparung (39) für den Durchtritt des Arms angebracht ist.

15. Orthopädische Weste nach einem der Ansprüche 1 bis 13, dadurch gekennzeichnet, dass das zweite Feld (3) und das dritte Feld (4) nur bis in Höhe des vorderen Arms fest miteinander verbunden sind und dass zusätzliche Befestigungsmittel (21, 22) vorgesehen sind, mittels derer die Felder (3 und 4) in Höhe der Unterseite des Arms miteinander verbindbar sind.

16. Orthopädische Weste nach Anspruch 15, dadurch gekennzeichnet, dass zusätzliche Befestigungsmittel (23) vorgesehen sind, die die Benutzung der Weste als Schienungsmittel erleichtern.

17. Orthopädische Weste nach einem der Anspüche 1 bis 16, dadurch gekennzeichnet, dass wenigstens ein Teil der Befestigungsmittel (8 bis 19) in horizontaler Richtung angeordnet sind.

18. Orthopädische Weste nach einem der Anspüche 1 bis 16, dadurch gekennzeichnet, dass wenigstens ein Teil der Befestigungsmittel (8 bis 19) in vertikaler Richtung angeordnet sind.

19. Orthopädische Weste nach einem der Anspüche 1 bis 16, dadurch gekennzeichnet, dass wenigstens ein Teil der Befestigungsmittel (8 bis 19) in geneigter Richtung angeordnet sind.

20. Orthopädische Weste nach einem der Anspüche 1 bis 19, dadurch gekennzeichnet, dass die Verbindung- und Befestigungsmittel nach Art von Klettverschlüssen ausgebildet sind, d.h. solchen, deren Verbindungspartner aus einem Schlingenstoff bzw. aus Haken bestehen.

21. Orthopädische Weste nach Anspruch 20, dadurch gekennzeichnet, dass ein zusätzliches Befestigungsmittel aus Schlingenstoff (20) vorgesehen ist, das mit auf der Oberfläche der Weste erscheinenden, aus Haken bestehenden Befestigungsmitteln zusammenarbeiten kann.

22. Orthopädische Weste nach einem der Ansprüche 1 bis 21, dadurch gekennzeichnet, dass ein äusseres oder inneres Ärmelsystem (41) vorgesehen ist, das ein eigenes Umwende- und Spannsystem (42) besitzt.

23. Orthopädische Weste nach einem der Ansprüche 1 bis 22, dadurch gekennzeichnet, dass die Armschlinge mehrere Befestigungsmittel besitzt, die derart nebeneinander angeordnet sind, dass die Armschlinge an den Umfang des zu haltenden Arms anpassbar ist.

24. Orthopädische Weste nach einem der Anspüche 1 bis 23, dadurch gekennzeichnet, dass in der Armschlinge ein Schlitz (28) für den Austritt der Hand angebracht ist.

**Claims**

1. An orthopaedic jacket to support and restrain the shoulder, the scapular girdle and the corresponding upper limb, comprising:
– a first panel (2) intended to cover the rear portion of the thorax of the wearer of the jacket,
– a second panel (3) which extends the first panel (2) laterally and is intended to cover the front portion of the thorax, the axillary region and the rear portion of the shoulder and of the arm to be supported,
– a third panel (4)
– a forth panel (5) which partially extends the lower portion of the second panel (3) and is intended to act as a support for the forearm of the wearer of the jacket,
– means of closing and progressive thightening provided on both faces of the said panels enabling the jacket to be used indifferently on the left or right-hand side merely by turning it over,
– an opening (38, 39) provided between the second and third panels (3, 4) for passage of the affected arm, characterized in that
– the second panel (3) is so shaped as to cover by itself practically the whole of the front portion of the thorax of the wearer of the jacket,
– the third panel (4) extends the second panel (3) on the side opposite to the first panel (2), and in that
– the third panel (4) is so shaped as to be able to contain the rear portion of the shoulder and the affected arm, and to cover at least a part of the first panel (2) to hold it in place on the rear portion of the thorax, thus ensuring closure of the jacket in the small of the wearer's back.

2. An orthopaedic jacket according to Claim 1, characterized in that the first panel (2) is approximately rectangular and is laterally extended by the second panel (3), which is bounded as regards its lower portion by a line (30), approximately horizontal, extending the lower edge of the first panel; as regards its upper portion by arising concave curve (31), and, as regards the side opposite to the first panel, by a convex descending curve (32) extended by a line that is approximately vertical (33), the second panel being joined along this line by a seam or other suitable means to a third panel (4) the assembly edge of which offers the same profile, the said third panel in other respects being bounded in its upper portion by an oblique line (34), in its lower portion by an approximately horizontal line (35) extending the lower edge of the first and second panels, and at its free edge by an approximately vertical line (36), and that the fourth panel (5) has a shape that is approximately rectangular and is joined along one of its sides to the lower portion of the second panel.

3. An orthopaedic jacket according to Claims 1 and 2, characterized in that the seam-line between the second and third panel is interrupted in its lower part, thus providing a slit permitting passage of the forearm.

4. An orthopaedic jacket according the Claims 1 and 2, characterized in that the seam-line between the second and third panel is interrupted, for the purpose of providing a circular cut on both sides of this interruption, enabling an opening (39) to be produced, permitting passage of the forearm.

5. An orthopaedic jacket according to any one of Claims 1 to 4, characterized in that panels (2), (3) and (4) forming the body of the jacket (1) extend as far as the wearer's waist and the arm-support is joined to panel (3) at the level of a slit (38) provided in the said panel (3) above the waist.

6. An orthopaedic jacket according to Claim 5, characterized in that means of fixing are provided in the lower portions of panels (2), (3) and (4) to work in conjunction with complementary means provided on the trousers or the belt.

7. An orthopaedic jacket according to any one of Claims 1 to 6, characterized in that it is equipped with a detachable or semidetachable tongue (6), supplied with its own fixing means (12) and (13) provided for use in conjunction with other means (14) and (15) placed on the said jacket, enabling support and protection of the under-part of the elbow and retention of the forearm within the jacket to be ensured.

8. An orthopaedic jacket according to any one of Claims 1 to 7, characterized in that it is equipped with another detachable or semi-detachable tongue (7) situated at the level of the shoulder where it is retained by its own means of attachment (16) and (17) enabling on one hand traction of the shoulder towards the rear with greater or lesser force to be exerted as required, and on the other hand enabling the shoulder to be maintained in a position of external rotation due to adjustable fixation means (18) and (19) provided on the said jacket and capable of working in conjunction with the said tongue's own means of attachment, these means being located in positions facilitating their setting.

9. An orthopaedic jacket according to any on of Claims 1 to 8, characterized in that the different panels forming the body of the jacket (1) are made of material that can in itself exert a certain amount of restraint.

10. An orthopaedic jacket according to Claim 9, characterized in that it is made of an elastic fabric or knittes material.

11. An orthopaedic jacket according to Claim 9, characterized in that it is composed of an assembly of impermeable sheets joined to one another in water-tight manner and that it can be inflated by the introduction of a fluid.

12. An orthopaedic jacket according to Claim 9, characterized in that it is conposed of an assembly of impermeable sheets and can be inflated by a depression system.

13. An orthopaedic jacket according to Claims 7 and 8, characterized in that the tongues are made of an elastic material, possibly of the same composition as that forming the body of the jacket.

14. An orthopaedic jacket according to any one of Claims 1 to 13, characterized in that the seamline provided between panels (3) and (4) is interrupted at a level below the arm for the purpose of providing a circular cut (39) on both sides permetting passage of the arm.

15. An orthopaedic jacket according to any one of Claims 1 to 13, characterized in that panels (3) and (4) are only joined together as far as a level below the arm, and complementary means of fixing (21) and (22) are provided to join panels (3) and (4) together at the level below the arm.

16. An orthopaedic jacket according to Claim 15, characterized in that cupplementary means of fixing (23) are provided to assist the use of the jacket as spling wrapping.

17. An orthopaedic jacket according to any one of Claims 1 to 16, characterized in that at least part of attachment means (8), (9), (10), (11), (12), (13), (14), (15), (16), (17), (18) and (19) are arranged in a horizontal direction.

18. An orthopaedic jacket according to any one of Claims 1 to 16, characterized in that at least part of attachment means (8), (9), (10), (11), (12), (13), (14), (15), (16), (17), (18) and (19) are arranged in a vertical direction.

19. An orthopaedic jacket according to any one of Claims 1 to 16, characterized in that at least part of attachment means (8), (9), (10), (11), (12), (13), (14), (15), (16), (17), (18) and (19) are arranged in an oblique direction.

20. An orthopaedic jacket according to any one of Claims 1 to 19, characterized in that the means of attachment and fixation are of the velour and hooks type.

21. An orthopaedic jacket according to Claim 20, characterized in that complementary means of the velour type (20) are provided to co-operate with the means of hook type appearing on the surface of the jacket.

22. An orthopaedic jacket according to any one of Claims 1 to 21, characterized in that it comprises a sleeve system (41), outside or inside the jacket, comprising its own system of reversibility and fastening together (42).

23. An orthopaedic jacket according to any one of Claims 1 to 22, characterized in that the arm support is equipped with several means of fastening (11) juxtaposed in such a way as to allow adaptation to the size of the arm to be supported.

24. An orthopaedic jacket according to any one of Claims 1 to 23, characterized in that a slit (28) is provided in the arm support (5) to enable the hand to emerge.

FIG_1

FIG_2

0077345

FIG_3

FIG_4

FIG_5

13

0077345

FIG.6

FIG.7

FIG.8

FIG.9

15

FIG_10

FIG_11

FIG_12